# EUROPEAN PATENT APPLICATION

(11) **EP 4 671 378 A1**
(43) Date of publication of application: **31.12.2025**
(21) Application number: 24185172.4
(22) Date of filing: 27.06.2024
(51) Int. Cl.: C12Q 1/00, A61B 5/00, C12Q 1/26, C12Q 1/28, C12Q 1/34, C12Q 1/44, C12Q 1/46, C12Q 1/48, G01N 27/327

(54) **BIOELECTRONICS BASED ON FUNCTIONALIZED PROTEINS**

(71) Applicant: Vilnius University, 01513 Vilnius (LT)
(72) Inventor: Bagdziunas, Gintautas, LT- 10257 Vilnius (LT); Ragauskaite, Elzbieta, LT- 10257 Vilnius (LT); Macaityte, Diana, LT- 10257 Vilnus (LT); Dudkaite, Vygaile, LT- 10257 Vilnius (LT); Mikenaite, Greta, LT- 10257 Vilnius (LT); Vecelyte, Guoda, LT- 10257 Vilnius (LT); Tetianec, Lidija, LT- 10257 Vilnius (LT)
(74) Representative: AAA Law

(57) **Abstract**

The invention relates to chemical, electrochemical and non-covalently bioconjugation (functionalization) of proteins with catalytic properties. A functionalization method is disclosed using all organic solvents except dimethylformamide (DMF), and dimethyl sulfoxide (DMSO), and utilizing organic synthesis and non-covalent insertion methods. Furthermore, the invention encompasses the preparation of bioelectrodes designed for the detection of substances, including but not limited to glucose, lactose, galactose (as carbohydrates), dopamine, adrenaline (epinephrine), noradrenaline (norepinephrine), and serotonin (as neurotransmitters), L-glutamate, D- or L-amino acids, hydrogen peroxide, nicotine, cholesterol, ethanol, oxalate, lactate, and pyruvate in food products, as well as in human body fluids serving as real samples. Additionally, this invention extends to the utilization of bioelectrodes for monitoring chemical processes, including features and the chemical environment of neuron interfaces within the brain. The invention encompasses an electrochemical inhibitor testing system utilizing the aforementioned bioelectrodes, and the functionalized proteins as single-molecular protein-based memristors.

## Description

### TECHNICAL FIELD

The invention relates to bioelectronics based on chemical, electrochemical, and non-covalently bioconjugated (functionalized) proteins with catalytic properties using redox-active groups. The functionalized proteins are prepared in organic solvents and used in bioelectronics, such as electrochemical biosensors for the concentration measurement of bioanalytes in clinical and food applications. These functionalized proteins can also be used in enzyme-based logic gates, memristors, and biosensors based on enzyme inhibition for clinical and pharmaceutical applications, as well as in brain-computer interfaces.

### BACKGROUND ART

Direct charge carrier (hole or electron) transfer is an important factor in biological systems that undergo oxidation or reduction processes. By copying and improving the processes of living nature, this transfer can be applied in bioelectronics. The protein functionalization technology relies on the direct electron transfer (DET) phenomenon between bioconjugated proteins and electrodes. DET is an electron transfer process where the electrons are tunnelled from the electrode to the redox cofactor of a protein which is a protein having catalytic properties.

Amperometric enzymatic biosensors are divided into three generations based on the principle of analytical signal registration:
- the first-generation biosensors rely on electron transfer from the cofactor of enzyme, which is immobilized on a working electrode, to molecular oxygen as the electron acceptor. This process produces hydrogen peroxide as a reduced product, which is then detected electrochemically;
- in the second generation, artificial redox mediators in an electrolyte are used to facilitate electron transfer. However, the ultimate goal of biosensor development is to eliminate the use of mediators, reducing fabrication costs and complexity while enhancing durability. This leads to:
- the third generation of biosensors, which leverage direct electron transfer (DET) from the immobilized enzyme to the working electrode, offering a more advanced sensing approach.

By combining second- and third-generation biosensors, a new type of an enzyme with non-covalently included and covalently attached electron mediators - also known as wired enzymes - can be created. To achieve this, a part of the shell of the enzyme must be either stripped or modified with redox-active or semiconducting compounds that act as electron relays, making the enzyme electrically conductive. These early enzyme modification attempts involved glucose oxidase enzymes bioconjugated with ferrocene, as disclosed in the documents **[1] [2] [3],** and other redox mediators as disclosed in the documents **[4] [5]** to create wired enzyme-based biosensors. However, these biosensors did not demonstrate the desired characteristics for practical applications, primarily due to low solubility of the precursors of the mediators in aqueous solutions.

To address this problem, we have demonstrated in our previous research **[6],** that glucose oxidase, a protein with catalytic properties, can remain stable in organic solvents. However, we have also discovered that polar organic solvents like dimethyl sulfoxide (DMSO) and dimethylformamide (DMF) can strongly coordinate with the amide bonds in the amino acid residues at the active center, preventing a substrate binding. Additionally, we have tested chloroform as a medium for bioconjugating glucose oxidase, finding that it is not effective due to the formation of a stable layer of water molecules onto the surface of protein, as disclosed in **[7].**

The WO2018062542A1 invention relates to glucose oxidoreductase bioconjugated with phenazine derivative as a novel electron mediator-modified protein that is excellent in electron transfer efficiency and enables highly sensitive and highly accurate measurement of biological materials, an electrode using the redox mediator-modified enzyme, and a spectroscopic analysis kit and enzyme test paper.

The CN110511913B invention relates to a mutant glucose oxidase and use thereof. An electron acceptor modified glucose oxidase is obtained by using a mutant glucose oxidase in which an amino acid residue corresponding to isoleucine 489 or arginine 335 of the amino acid sequence is replaced with an amino acid residue having a reactive functional group in a side chain, and binding an electron acceptor with the amino acid residue having a reactive functional group.

The US2010/0316643A1 invention provides novel targeted antimicrobial compositions. In various embodiments chimeric moieties are provided comprising an antimicrobial protein attached to a protein targeting moiety that binds a bacterial Strain or species. In this invention, functionalized proteins with water soluble dyes and organic compounds were applied as antibiotics. All the procedures were performed in aqueous solutions.

The European patent EP2535703B1 and its related international patent application WO2009042631 A2 disclose biosensor systems including a measurement device and test sensors including at least three independently addressable electrodes, with at least two of the electrodes being substantially chemically isolated are disclosed. One or more working electrodes are combined with two or more counter electrodes. The two or more counter electrodes may operate at different potentials to provide for multi-analyte electrochemical analysis. Analysis methods are provided to perform multi-analyte electrochemical analysis and test sensors are provided having resistance to chemical mixing between secondary analysis regions. In this invention, native (not functionalized) enzymes were used as the recongation element in the measurement device and test sensors.

One more US patent US8691073B2 describes an electrochemical sensor strip having a base and a first electrode and a second electrode on the base. An oxidoreductase enzyme and a mediator are on the first electrode, and a soluble redox species is on the second electrode. The soluble redox species may be an organotransition metal complex, a transition metal coordination complex, an electroactive organic molecule, or mixtures thereof. In this type of glucose sensor was developed, in which native glucose oxidase and an organic compound such as potassium ferricyanide, a ferrocene derivative and a quinone derivative or a metal complex was used as an electron acceptor instead of oxygen in the aqua solution.

The above overviewed closest documents disclose bioelectronics such as biosensors based on glucose oxidoreductase bioconjugated with phenazine derivative, a mutant glucose oxidase for multi-analyte electrochemical analysis, an antimicrobial protein attached to a protein targeting moiety, biosensors with three independently addressable electrodes, and with a soluble redox species. They disclose functionalization of proteins for biosensing applications, using water and chloroform solutions. Furthermore, only certain proteins are disclosed to detect certain analytes, while the demand is for biosensing of multiple analytes in a mixture thereof.

### SUMMARY OF INVENTION

**Technical problem.** Bioconjugation of proteins enhances their functional diversity by manipulating their structure and function. These bioconjugates play a crucial role in exploring significant information about protein interactions with small molecules and serving as the selectivity-generating components in biosensors. It is widely acknowledged that biosensors relying on direct electron transfer (DET) between enzyme cofactor and electrode offer the most efficient and selective detection of bioanalytes. However, enzymes capable of DET are either scarce only a few examples are known such as FAD and pyrroloquinoline quinone (PQQ)-glucose dehydrogenases, fructose dehydrogenase, fructose dehydrogenase, P450 cytochromes, and myoglobin.

We suggest that both holes and electrons can be transferred through the aromatic amino acid residues of a native enzyme, with DET estimating this transfer distance to be around 1 nm **[8].** According to our theoretical study, DET should be facilitated by easily oxidizable groups acting as electron mediators on the enzyme surface. However, it has become apparent that enzymes often cannot exchange electrons with the electrodes on which they are adsorbed because their active sites are electrically inaccessible. This limitation can make native proteins and enzymes challenging to apply in bioelectronics. Consequently, functionalizing proteins with redox groups as semiconducting mediators having catalytic properties emerges as an excellent strategy for obtaining biostructures with such capabilities. However, most organic semiconductor (redox) mediators are poorly soluble in water but soluble in organic solvents. Therefore, our solution to this technical problem is peptide/protein/enzyme modification in organic solvents where the dissolved proteins remain stable.

**Solution.** The present invention aims to solve the above specified technical problem in bioelectronics technology by introducing a novel and efficient (1) method for modifying (2) proteins with catalytic properties using (3) electrochemically (redox) active groups in (4) organic solvents (except chloroform, DMF, and DMSO). These groups facilitate the flow of electrons from the electrode to the cofactor of the protein or *vice versa.* The previously by our earlier research disclosed protein stability, i.e., the protein retains its original structure and function, in organic solvents provides a basis for the application of redox-functionalized proteins in biosensors. This invention expands the possibilities for using bioconjugated proteins with catalytic properties in bioelectronics such as biosensors, electrochemical inhibitor testing system, and memristor. The bioconjugated proteins can be easily immobilized onto electrodes and adapted to multi-electrode systems with a possibility for operating them at a single working potential, since their working potential depends on the used redox function group as semiconducting mediator. Furthermore, an essential consistent part of the present invention is variety of functionalised proteins obtainable by the aforementioned method, thereby retaining at least 30% or more of their biocatalytical activity compared to the native protein.

The solution aims to functionalize biosensitive proteins with substituted functional groups when precursors of the functional groups to be attached to the protein are water-insoluble or slightly-soluble (in the range of 0.1 to 1.0 mg/mL), under the limitation that the biosensitive protein is stable in an organic solvent, and said precursor of the functional group is soluble in said organic solvent. Thus the biosensitive protein is functionalized with a substituted functional group in said organic solvent. The functionalization process is done without a dialysis.

**Novelty.** The solution presented is the boconjugation of proteins within organic solvents (except chloroform, DMF and DMSO) using redox-active and organic semiconducting groups. These redox-active functional groups can be attached to the surfaces of protein using conventional organic chemistry methods, such as: condensation, organic electrochemical synthesis, and cross-coupling, reductive amination, and nucleophilic, or electrophilic substitution reactions. Furthermore, this approach allows for the non-covalent insertion of redox-active groups, acting as mediators, into the protein structure during preparation in organic solvents. The combination of a layer of the functionalized proteins with the redox-active group alongside a layer of the corresponding protein onto an electrode, can also find application in biosensors for detecting and monitoring bioanalytes in clinical and food applications and the chemical environment of neuron interfaces within the brain, electrochemical inhibitor testing system, and memristor for neuromorphic computing. These bioconjugated proteins can be attached to electrode surfaces without the use of the layers of other materials. This approach allows the multiple operational bioelectrodes to be employed in a single biosensor, enabling the detection of numerous relevant analytes in one sample.

**Technical effects.** The present invention offers several advantageous effects in addressing to solution of the technical problem:
**Effect 1:** Functionalizing proteins with catalytic properties in polar and apolar organic solvents (except chloroform, DMF and DMSO) proves to be an efficient and rapid method for obtaining redox-active biorecognition elements for bioelectronics. This process results least 30% or more of their biocatalytical activity compared to a native protein and a similar activity compared to the approach by functionalization in water. This approach in organic solvents eliminates the need for complex chromatographic purification methods. Consequently, it provides a simple production (bioconjugation) approach. All the organic semiconducting or redox (T2-1) ferrocene, (T2-2) carbazole, (T2-3) phenothiazine, (T2-4) phenoselenazine, (T2-5) phenoxazine, (T2-6) acridane, (T2-7) thianthrene, (T2-8) selenanthrene, (T2-9) anthraquinone, (T2-10) fullerene, (T2-11) perylene tetracarboxylic diimide derivatives are highly soluble in organic solvents, but poorly soluble in water. The process of functionalizing all the types of proteins and fabricating the different type bioelectrodes follows a same approach. As a result, these production steps can be easily automated, also for different types of catalytic proteins and biolectrodes.
**Effect 2:** The bioelectrodes based on the biorecognition elements produced through this method can function under the same operational conditions, including applied electric potential, salinity, and pH. The self-assembly monolayer of the functionalized proteins and their mixture with non-funcionalized enzymes can be deposited onto the bioelectrode. This uniformity allows the multiple-type operational bioelectrodes with different immobilized specific functionalized proteins to be employed in a single biosensor, thereby enabling to detect more than one different relevant analytes in a single sample under test. This allows to use multiple different electrodes *in vivo* environments, for example, bioelectrochemical brain interface.
**Effect 3.** The electrochemical biosensor can comprise several different electrodes thereby forming a combined biosensor with more than one electrode is contained in a single device. Such combined biosensors can operate at a low potential between - 0.2 V and 0.5 V using an Ag/AgCI reference electrode. For the DET-based bioelectrodes, the linear relationship between current and concentration is not constrained by the levels of analyte and oxygen present in the electrolyte. Consequently, they do not exhibit a response to extraneous compounds such as ascorbic acid and uric acid due to this low working potential.
**Effect 4.** The biosensors based on enzyme inhibition, using immobilized functionalized enzymes, work by measuring enzymatic activity with and without an inhibitor to quantify their concentration. This electrochemical bioelectrode system is designed for rapid inhibitor determination by testing sequentially many potential inhibitors in a single pot.
**Effect 5.** The computer-brain interface using invasive biosensors based on the functionalized enzymes can convert neurochemical responses into electrical signals. This technology monitors brain responses by recording changes in the concentration of neuroanalytes, such as glucose, lactose, or neurotransmitters, in real time. The multiple operational bioelectrodes with the immobilized specific functionalized proteins can be employed in a single biosensor.
**Effect 6.** The bimodal memristor and their logic element powered by the substrate or protons based on self-assembled monolayer of the functionalized proteins onto a surface. In this case, logical elements can be constructed using a single functionalized protein as a unit element. In these logic elements, the current-voltage (I-V) characteristics or ON/OFF states change depends on the low oxidation potential of a redox mediator and concentration of substrate of the functionalized protein.

### BRIEF DESCRIPTION OF DRAWINGS

The features and advantages of the invention are described in the detailed description of the invention concerning the drawings below:
- **Figure 1**: The electrochemically active various functionalized (T2-1) ferrocene, (T2-2) carbazole, (T2-3) phenothiazine, (T2-4) phenoselenazine, (T2-5) phenoxazine, (T2-6) acridane, (T2-7) thianthrene, (T2-8) selenanthrene, (T2-9) anthraquinone, (T2-10) fullerene, (T2-11) perylene tetracarboxylic diimide derivatives used for the functionalization of the proteins:
- **Figure 2**: Schematic presentations of two types of bioelectrodes:
**a)** the invented bioelectrode based on based on a self-assembled molecular monolayer (SAM) of functionalized peptide;
**b)** the bioelectrode based on SAM of functionalized peptide with a deposited layer of non-functionalized peptide;
- **Figure 3**: The principal scheme of the biosensor system containing more than one working bioelectrode.
- **Figure 4**: The electrochemical properties of the bioelectrode based on SAM of the functionalized functionalized glucose oxidase (GOx):
**a)** bioelectrocatalytic currents by CV after addition of 100 mM of glucose;
**b)** the increase in currents by increasing glucose concentration (in the inset: data of liner fitting and the change in currents at glucose concentrations);
**c)** the chronoamperometry by adding interfering reagents that may be present in human body fluids or food samples.

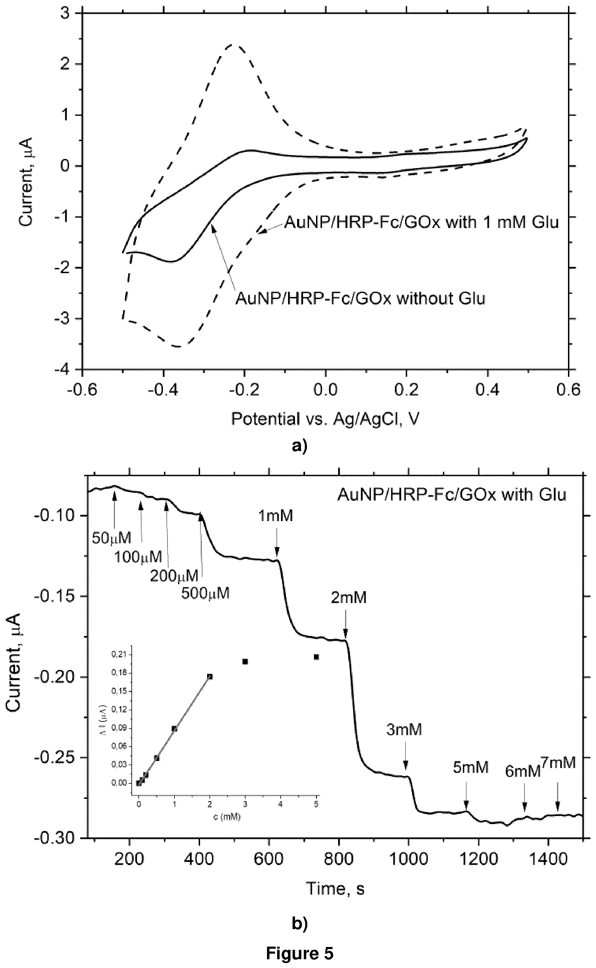

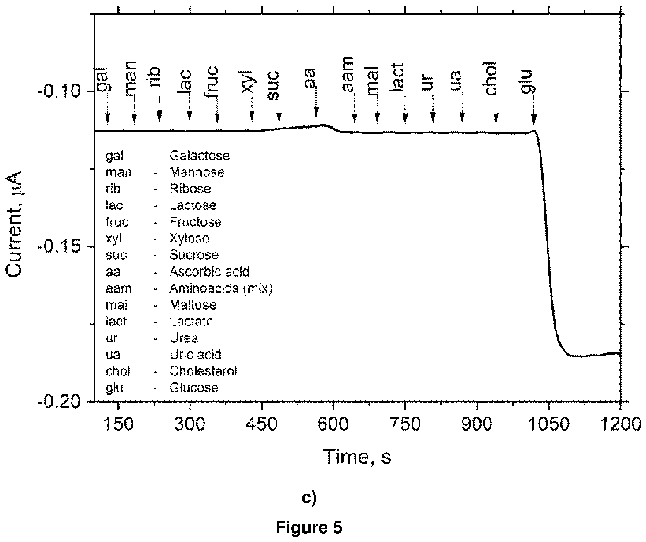

- **Figure 5**: The electrochemical properties of the bioelectrode based on SAM of the functionalized horseradish peroxidase (HRP-Fc):
**a)** currents by CV after the addition of 1 mM of glucose;
**b)** the increase in currents by increasing glucose concentration (in the inset: data of liner fitting and the change in currents at glucose concentrations);
**c)** the chronoamperometry by adding interfering reagents that may be present in human body fluids or food samples.

### TERMS AND ABBREVIATIONS OF THE INVENTION

- AuNP: gold nanoparticle;
- CV: cyclic voltammetry;
- DET: direct electron transfer;
- DMF: dimethylformamide;
- DMSO: dimethyl sulfoxide;
- FAD: flavin adenine dinucleotide;
- GOx: glucose oxidase enzyme;
- SAM: self-assembled molecular monolayer;
- HRP: horseradish peroxidase enzyme;
- PPB: potassium phosphate buffer;
- Protein: under the scope of this invention, the term "protein" is considered to be equivalent with other terms: "enzyme", "peptide"; most of proteins in this invention are enzymes, but several are not (e.g., myoglobin); further, "peptide" and "protein" make structural difference by length of the amino acids chain only;
- 1 U: enzyme unit is the enzyme activity that catalyses the conversion of 1 µmol substrate into product in 1 minute.
- Solubility: a substance is considered slightly soluble in water if its solubility falls into the range of 0.1 to 1.0 mg mL⁻¹.

### DETAILED DESCRIPTION OF THE INVENTION

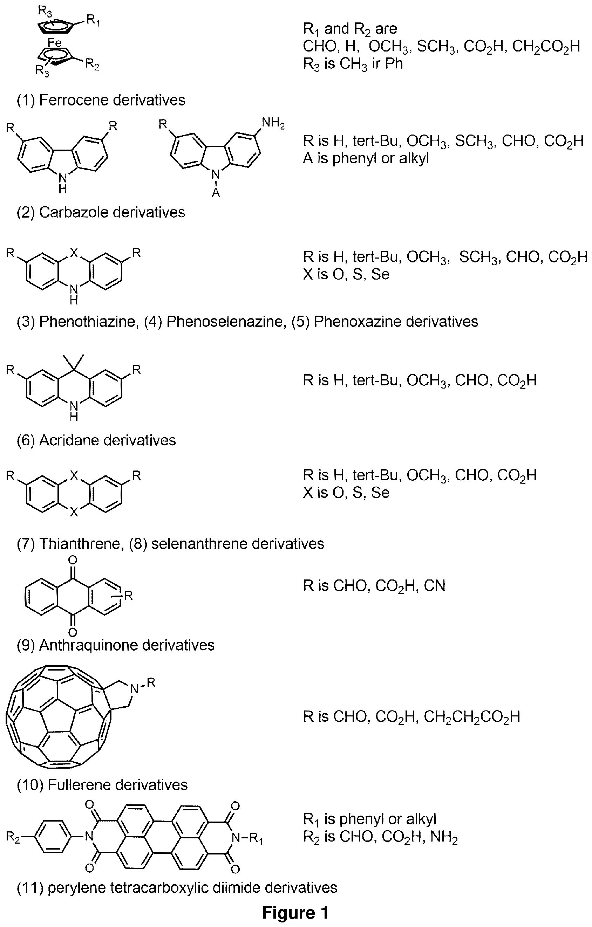

The present invention relates to functionalizing the proteins having catalytic properties thereby being suitable to detect the corresponding analytes (as presented in Table 1), with various substituted electrochemical active compounds (listed in Figure 1) and to use of these functionalized proteins in biosensors. Electrochemical biosensors that include the functionalized proteins as recognition elements were developed for the detection of analytes in biological, environmental and food samples.

**Table 1. Recognizing Proteins with Catalytic Properties and Their Analytes Available for Use.**

| **No.** | **Proteins with catalytic properties** | **No.** | **Analytes** |
|---|---|---|---|
| **T1-1** | Peroxidase from horseradish | **T1-1a** | Hydrogen peroxide, dopamine |
| **T1-2** | Glucose oxidase | **T1-2a** | Glucose |
| **T1-3** | Galactose oxidase and lactase | **T1-3a** | Galactose, lactose |
| **T1-4** | Cholesterol oxidase and cholesterol esterase | **T1-4a** | Cholesterol |
| **T1-5** | Alcohol oxidase | **T1-5a** | Ethanol, Methanol |
| **T1-6** | Monoamine oxidases A and B | **T1-6a** | Serotonin, adrenaline, noradrenaline, nicotine or other inhibitors of these enzymes |
| **T1-7** | Diamine oxidase | **T1-7a** | Adrenaline, noradrenaline |
| **T1-8** | GABase | **T1-8a** | γ-aminobutyric acid (GABA) |
| **T1-9** | D-Amine acid oxidase | **T1-9a** | D-amino acids, D-serine, D-lysine, serotonin |
| **T1-10** | L-Amine acid oxidase | **T1-10a** | L-amino acids |
| **T1-11** | L-Aspartate oxidase | **T1-11a** | L-aspartate |
| **T1-12** | L-Glutamate oxidase | **T1-12a** | L-glutamate, L-glutamide |
| **T1-13** | Lysine oxidase | **T1-13a** | L-lysine |
| **T1-14** | Glycerol-3-phosphate oxidase, glycerol kinase | **T1-14a** | Glycerol |
| **T1-15** | Coenzyme-A synthetase and acyl coenzyme-A oxidase | **T1-15a** | Free fatty acids, peroxisomal acetyl-CoA |
| **T1-16** | Acetylcholine esterase and choline oxidase | **T1-16a** | Acetylcholine, organophosphate insecticides, chemical weapon compounds as acetylcholine esterase inhibitors |
| **T1-17** | Oxalate oxidase | **T1-17a** | Oxalate |
| **T1-18** | Sarcosine oxidase | **T1-18a** | Sarcosine |
| **T1-19** | Xanthine oxidase | **T1-19a** | Xanthine |
| **T1-20** | Lactate oxidase | **T1-20a** | Lactate |
| **T1-21** | Pyruvate oxidase | **T1-21a** | Pyruvate |
| **T1-22** | Uricase | **T1-22a** | Uric acid |
| **T1-23** | Myoglobin | **T1-23a** | Oxygen, nitrogen(II) oxide |

The functionalization of proteins was carried out in aqua and organic solvents. First of all, the stability of the proteins in organic solvents was tested. Figure 1 shows the electrochemical active compounds applied to proteins as substitutes/functionalizers: (T2-1) ferrocene, (T2-2) carbazole, (T2-3) phenothiazine, (T2-4) phenoselenazine, (T2-5) phenoxazine, (T2-6) acridane, (T2-7) thianthrene, (T2-8) selenanthrene, (T2-9) anthraquinone, (T2-10) fullerene, (T2-11) perylene tetracarboxylic diimide derivatives (Fig. 1) used for the non- and covalently functionalization of the proteins. In the invention, the functionalization is understood as the non- and covalent attachment of functional groups to the protein structure. The functionalization of the proteins form Table 1 thereby implying amide, imine, amine hydrazone, and C-C bond formation in organic solvents was developed. Moreover, for the functionalization of the enzymes, an amperometric or potentiometric condition in organic solvents was employed. After the reaction, the organic solvent was evaporated at reduced pressure and room temperature. The obtained residue was dissolved in the corresponding buffer and the surplus of the used reagent was removed by extraction with chloroform. The resulting aqueous layer can be dialyzed or not.

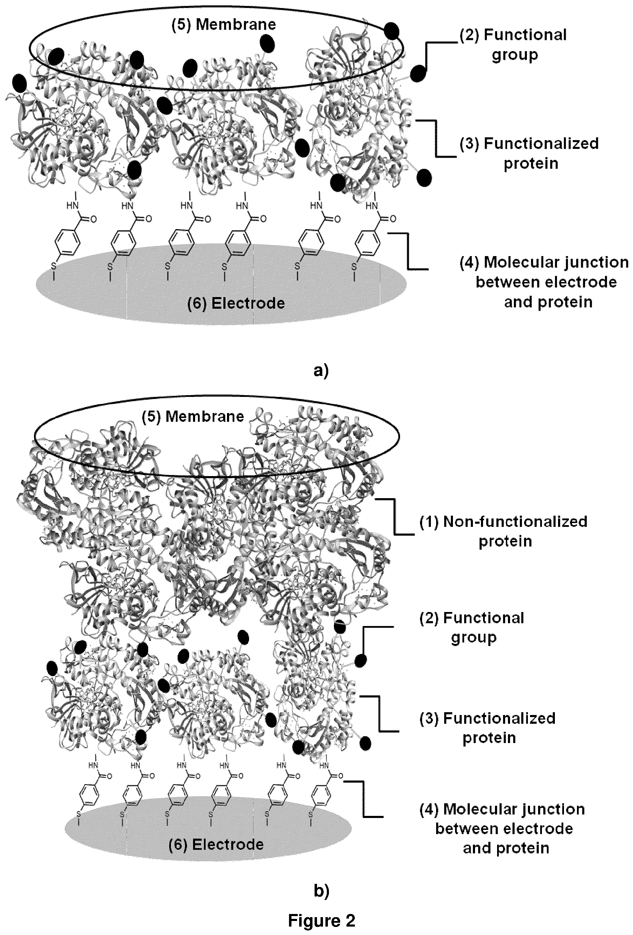

The present invention is based on the functionalized peptides immobilized onto conducting substrates with nanoparticles to make the direct charge carrier transfer-type (i.e., third generation) biosensors and bioelectrodes (Figure 2a). The SAM of the enzymes is the best method for immobilization of these enzymes on the electrode surface because the tunnelling distance (being up to 1 nm, as disclosed above, for DET) is a key factor in a direct charge carrier transfer-type bio-electrocatalysis.

**Comparative analysis of the invention.** To compare functionalization methods in aqueous and organic solvent, 3-(10*H*-phenoxazin-10-yl)propanoic acid (PPA) was conjugated to glucose oxidase (GOx) from *Aspergillus niger* as a source of functional groups. PPA is sparingly soluble in water (< 1 mg mL⁻¹) but highly soluble (> 1 mg mL⁻¹) in tetrahydrofuran (THF). The chemical reaction of amide bond formation was utilized for the functionalization by activating carboxylic acid groups of PPA with EDC, NHS, and HBTU in water and THF, respectively. After the synthesis and purification, the UV-vis spectra of functionalized GOx were recorded in a PPB pH 7 solution. Spectra of the unmodified GOx and initial PPA were also measured under the same conditions. Unmodified GOx showed absorption bands at 280 nm and 450 nm, while PPA displayed a distinct peak of light absorption at 325 nm. The spectra of functionalized GOx contained the characteristic absorption band of group of the initial compound (PPA). Deconvolution of the functionalized GOx spectra using the spectra of the initial compounds allowed for the estimation of attached groups. The number of attached groups was estimated to be 6.8 for functionalized GOx with PPA after bioconjugation in water and 6.2 after conjugation in THF. The X-ray structure of the GOx subunit (PDB: 3QVP) as disclosed in **[9]** reveals 12 lysine amino acid residues on the surface of one subunit. Therefore, approximately half of these residues were functionalized. Biocatalytic activities (units per mg of protein, U mg⁻¹) of native and functionalized GOx were examined using a coupled enzyme assay. In this assay, GOx oxidizes glucose to produce H₂O₂, which then reacts with horseradish peroxidase (HRP) and o-dianisidine (peroxidase substrate and colorimetric probe at 460 nm). The biocatalytic activities were estimated to be 70 U mg⁻¹ for the functionalized GOx with PPA after bioconjugation in water and 83 U mg⁻¹ after bioconjugation in THF. The biocatalytic activity of native GOx was 250 U mg⁻¹. These results suggest that functionalization in organic solvents can be effective for attaching low water-soluble organic compounds to peptides. The enzyme after these procedures retains approximately 30% of its biocatalytic activity compared to the native GOx. However, organic solvents remain a viable option for functionalization when water solubility of the modifying agent and reagents is a limitation. After dialysis purification, no significant changes in the purity and activity of the functionalized protein were observed. In the present experiment with GOx, the biocatalytic activity was retained at not less 30%. In case of functionalising other proteins from Table 1, this ratio of efficient biocatalytic activity between functionalized and native protein can vary from 10% and more. For the general scope of the 23 proteins, their biocatalytic activity compared to the corresponding native proteins is at least 10%, preferably 15%, more preferably 20%, and more preferably 25%, and most preferably at least 30%.

***In vivo* screening.** In medical applications, early screening for bio-analytes present *in-vivo* body fluids is crucial for the prevention and management of various diseases. Developing low-cost methods and tools for diagnosing infectious diseases and biomarkers of other conditions is a growing challenge critical to improving public and personal health. Electrochemical biosensors, which combine bioreceptors and transducers based on the wired enzymes to convert biological responses into electrical signals at a single applied potential, are keys to achieving fast and accurate biosensing of several analytes in a single sample. The present invention provides methods and compositions for biosensors designed to detect several target analytes from real samples, including milk, saliva, blood serum, brain tissue, as well as from food and industrial products.

**Biosensors based on enzyme inhibition.** This invention introduces biosensors based on enzyme inhibition. Utilizing the biosensors, inhibitors of a specific enzyme can be identified as potential biologically active substances. Enzyme inhibition occurs when a specific inhibitor interacts with an enzyme, affecting its functional groups and/or active sites in a way that significantly reduces its normal catalytic activity. Biosensors that operate based on enzyme inhibition work by measuring the enzymatic activities in conditions of the absence and the presence of an inhibitor, and then quantifying the inhibitor concentration with the substrate in the electrolite of the corresponding inhibited enzyme. The biosensors based on functionalized peptide showed good sensitivity, which allowed the determination of micro-molar concentrations of inhibitors, as well as the investigation of compounds that can be potential inhibitors of human monoamine oxidases A and B and acetylcholine esterase and choline oxidase, xanthine oxidase. The biosensor based on human monoamine oxidases exhibited higher sensitivity to lower nicotine concentrations and was therefore applied to the detection of real nicotine samples of unknown concentration that were obtained from smoked tobacco or other products. Insecticides, chemical weapon compounds, and potential antidepressants can be tested using this biosensor.

**Bioelectrochemical computer-brain interface.** A type of a bioelectrochemical computer-brain interface is an interface using invasive biosensors with the functionalized proteins and enzymes immobilized / deposited onto electrodes, that can convert neurochemical responses from cerebral fluids and neuroanalytes such as hydrogen peroxide, neuromediators, glucose, L-lactate, and L- and D-amino acids into electrical signals, that further are transferred to a computer for further analysis. Bioelectrodes provide high temporal resolution for detecting and measuring brain activity at a low working potential, which does not damage brain tissue and ensures good analyte selectivity. The bioelectrodes uniquely integrates *in vitro* and *in vivo* approaches, providing a comprehensive understanding of dynamics biomarker of the mental illness such as Alzheimer's and Parkinson's diseases. This combination of methodologies allows for a nuanced exploration of biomarker behaviour in both simulated and real physiological conditions.

**Bimodal memristors.** For mimicking neuromorphic computing systems, chemical information is transformed to electrical signals. Protein materials enable bimodal memristor operation via voltage and concentration of substrate as well as protons, and functionalization of peptides extends the controllability of the memristor. This invention introduces chemoelectronic memristor based on single-molecule bioconjugated (wired) proteins with catalytic properties. In these biodevices, the current-voltage (I-V) characteristics or ON/OFF states change depending on the potential of cofactor or functional group and substrate of the enzyme, such as glucose, hydrogen peroxide, oxygen, or neuromediators. The ultimate goal is to design a microfluidic lab-on-a-chip capable of performing multi-enzyme-catalyzed cascades, operating similarly to an electronic chip. The logical elements can be constructed using a single functionalized protein as a unit element, which is immobilized onto the substrate as a monolayer. This chip aims to integrate large networks arranged for detecting and processing chemical signals with electronic components, and facilitate integration of such biochemoelectrosensor chip with the brain or neuromorphic computing. This invention enables the production of bioelectronics at the single molecule level using biotechnological and protein bioconjugation methods.

**The proteins immobilized by a step-by-step method.** First, 4-mercaptobenzoic acid (MBA) is used to deposit the monolayer onto the bioelectrode surface.

Secondly, carboxylic acid groups of this acid on the bioelectrode surface were activated using 1-ethyl-3-(3-dimethylaminopropyl) carbodiimide (EDC) and N-hydroxy-succinimide (NHS) as the classical amide bond forming and the immobilization reagents. The bioelectrodes were immersed into corresponding functionalized enzyme solutions to obtain the SAMs of the enzymes onto the direct charge carrier (hole or electron) transfer-type bioelectrode (Figure 2a).

The flavin adenine dinucleotide (FAD) or copper cluster-based enzymes were deposited on the SAM of this functionalized horseradish peroxidase enzyme (HRP) to obtain the hydrogen peroxide detecting bioelectrode (Figure 2b). The present invention also relates to a monolayer of ferrocene functionalized peroxidise from horseradish (HRP-Fc) that was used to detect hydrogen peroxide and as the first-generation biosensors employing a layer of the native enzymes from Table 1. Other hydrogen peroxide-generating enzymes (Table 1) were loaded onto this HRP-Fc bioelectrode by achieving selectivity for specific analytes. Moreover, the sensitivity of the bioelectrodes is improved using the mixtures of oxidase and hydrolase (esterase) such as cholesterol oxidase and cholesterol esterase, galactose oxidase and lactase, as well as acetylcholine esterase and choline oxidase, respectively. This bioelectrode structure is presented in Figure 2b.

Another aspect of the present invention is providing a method of making electrochemical biosensors based on these bioelectrodes. The electrochemical biosensor comprised of a working electrode based on gold, platinum, gold, silver, copper, aluminium, iridium, palladium, platinum, rhodium, silicon, zinc, iron, steel, indium tin oxide and fluorine-doped tin oxide glass, graphite, and graphene conducting substrate with or without the gold (Au), silver (Ag), platinum (Pt), alumina (Al₂O₃), zinc oxide (ZnO) and silica (SiO₂) nanoparticles, as well as a porous coating disposed on at least a portion of the polymeric film. An auxiliary electrode and/or reference electrode are used to construct the biosensors.

Incorporation of nanoparticles in at least some instances improves sensitivity. A protective porous, thin coating may improve the long-term stability of the bioelectrode, suppress or eliminate interference from other electroactive species and/or reduce fouling or degradation of the bioelectrode. Metallic nanoparticles are also a component of the bioelectrode. The presence of nanoparticles increases the surface area of the polymeric film and thereby increases the sensitivity and robustness of the electrode. The suitable nanoparticles include those made of gold (Au), silver (Ag), platinum (Pt), alumina (Al₂O₃), zinc oxide (ZnO) and silica (SiO₂). The nanoparticles have dimensions generally in the range of 5 nm to 100 nm.

The coating can be any porous polymeric material that protects the enzyme from mechanical impact and prevents from washing of the enzyme from the bioelectrode. In particular embodiments, the porous coating is an insulating or gel-type layer of about 5 to 50 nm in thickness and pores size from 5 nm to 400 nm. The polymeric materials such as polyethene terephthalate, Nafion, or cellulose film are used. The porous coating prevents or reduces the leaching of the enzyme from the bioelectrode. This may extend the functional life of the bioelectrode and allow consistent calibration for a longer period.

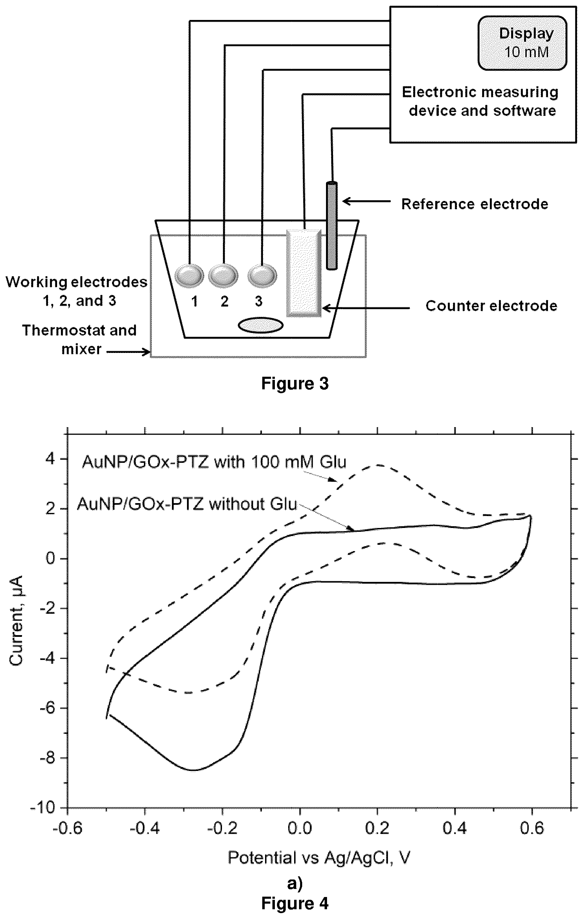

The principal scheme of the biosensor system with thermostat and magnetic mixer is presented in Figure 3. The reference electrode allows accurate measurement of potential at the working electrode to be made to a fixed reference potential. Any suitable reference electrode such as silver/silver chloride, mercury/mercury chloride, calomel, and platinum/hydrogen reference electrodes can be used. The working potentials of the biosensors are fixed to be 0.2 V for the direct charge carrier (hole) transfer type (Figure 2a) and -0.2 V vs. Ag/AgCI for the hydrogen peroxide detecting bioelectrode (Figure 2b). Also, a suitable counter electrode such as platinum wire, carbon fibre, glassy carbon, titanium plate or gold electrodes can be employed. In this three-electrode sensor, the potential of the working electrode is fixed relative to the reference electrode as a function of time. The applied potential serves as a driving force for the electron transfer reaction of the electroactive species on the bioelectrodes. The resulting current is a measure of the rate of the electron transfer reaction and is proportional to the concentration of the analyte.

### Experimental implementation of the invention.

Reagents. All chemicals were obtained from Sigma or Alfa-Aesar and were of analytical grade unless specified otherwise. The enzymes were obtained in lipolyzed form from Sigma. For all experiments, the enzymes were dissolved in a phosphate buffer (50 mM, pH 7.0) to 1.0 mg ml⁻¹ solution. The functionalized enzyme activity was measured photometrically according to Sigma assay procedures. Used organic solvents were supplied from Chempur and were distilled before use. Aqueous solutions were prepared with Milli-Q water. The bioelectrodes were tested in the solution of potassium phosphate buffer (PPB) (pH 7) and 100 mM KCI.

Instrumentation. Ultraviolet-visible (UV-Vis) spectra were measured to evaluate the kinetic constants using an Evolution 300 Security UV-Vis Spectrophotometer (Thermo Fisher Scientific). During the measurements, the samples were thermostated at 25 °C using an integrated thermostat. A JASCO J-815 Circular Dichroism spectrometer (Tokyo, Japan) was used to measure the circular dichroism (CD) spectra. The hydrodynamic diameter of nanoparticles was measured using Zetasizer µV (Malvern) and its compatible software.

**Preparation of the functionalized protein.** Methods of reductive amination (A), amide bond formation (B), and electrochemical functionalization (C) for the protein functionalization are presented:
Method A: 2 mg of the protein (Table 1), 200 molar equivalents of ferrocenecarboxaldehyde, and 2 mg of L-proline were dissolved in 2 mL of tetrahydrofuran or acetonitrile using ultrasound. Then, 2 mg of NaCNBHs was added to the solution. The mixture was stirred for 24 hours at 0 °C. The organic solvent was evaporated by rotary evaporator (the temperature of the bath should not exceed 40 °C) and the residue was dissolved in 2.0 mL of PPB buffer pH 7.0 solution. The solutions were filtered using membrane syringes (CA 0.45 µm) to remove not dissolved surplus of the starting compound. Additionally, the filtrate was extracted with 2 mL of chloroform, the upper layer was separated. After this chemical manipulation, the activity of the functionalized enzyme was photometrically tested.
Method B: The 200 molar equivalents of electrochemical active carboxylic acid by the protein, the same amount of 2-(1*H*-benzotriazol-1-yl)-1,1,3,3-tetramethyluronium hexafluorophosphate (HBTU), and 4 µL of diisopropylethylamine were added in round bottom flask and dissolved in 1 mL of tetrahydrofuran or acetonitrile using ultrasound. The mixture was stirred for 1 hour at room temperature. In this solution, 2 mg of the protein dissolved in tetrahydrofuran or acetonitrile was added. For a non-covalent attachment, the protein and organic compound were dissolved in the organic solvent in the same ratio. The mixture was stirred for 24 hours at room temperature. The organic solvent was evaporated by rotary evaporator (the temperature of the bath should not exceed 40 °C) and the residue was dissolved in 2.0 mL of PPB buffer pH 7.0 solution (or acetate, Tris, or citric buffer with various pH solution). The solutions were filtered using membrane syringes (CA 0.45 µm) to remove not dissolved surplus of the starting compound. Additionally, the filtrate was extracted with 2 mL of chloroform, the upper layer was separated. After this chemical manipulation, the activity of the functionalized enzyme was photometrically tested.
Method C: The solid compounds of 5 mg of the protein, and 200 molar equivalents of the electro-active compound were dissolved in 3 mL of the acetonitrile and 2 mL of PPB buffer with 0.1 M KCI using ultrasound. Using electrochemical cell containing an Ag/Ag⁺ reference electrode with 100 mM AgNOs, 1 M NEt₄BF₄ in acetonitrile and the graphite and titan plate electrodes, electrochemical reaction was carried out for 1000 s at 10 mA current under argon atmosphere, After the electrochemical reaction, the organic solvent was evaporated by rotary evaporator (the temperature of the bath should not exceed 40 °C) and the residue was dissolved in 2.0 mL of PPB buffer pH 7.0 solution (or acetate, Tris, or citric buffer with various pH solution). This solution was filtered using membrane syringes (CA 0.45 µm) to remove not dissolved surplus of the starting compound. Additionally, the filtrate was extracted with 2 mL of chloroform, the upper layer was separated. After this chemical manipulation, the activity of the functionalized protein was photometrically tested.

**Preparation of the bioelectrodes.** Methods of the electrode preparation and the immobilization of the functionalized proteins are presented:
Method A: For the immobilization, the working electrodes were firstly cleaned with Micropolish alumina 0.3 µm gel for 5 min. Then, the electrodes were cleaned with distilled water and put in an ultrasonic bath for another 5 min. Then, the electrodes were electrochemically cleaned. Subsequently, nanoparticles were added onto the electrode surface and left to dry or deposited by electrochemical methods. Once dried, the electrode is again cleaned via electrochemical method in 0.5 M H₂SO₄ solution. The electrodes were then washed with distilled water and inserted in Eppendorf tubes, which contained silane and amino groups, in an ethanol solution. Electrodes together with Eppendorf tubes were stored in a refrigerator at 4 °C for one hour. Then, the electrodes were washed in an ultrasonic bath for 2 min and transferred to other Eppendorf tubes, which contained 300 µL of the 2 mM 1-ethyl-3-carbodiimide hydrochloride (EDC) and 5 mM N-hydroxysuccinimide (NHS) solution or another coupling reagent and were kept at room temperature for 30 min. Afterwards, the electrodes were immersed in the 300 µL of the modified protein solutions in Eppendorf tubes to obtain a monolayer of the functionalized protein, which were tightly wrapped with parafilm tape and stored for 24-48 hours at a temperature of 4 °C.
Method B: Onto the bioelectrode prepared using this procedure of method B, 2 µL of solution (1 mg ml⁻¹ the protein in PPB) was deposited and evaporated or the drop is placed onto a layer of nylon material. A membrane was added onto the bioelectrode or crosslinked of this layer to prevent the protein from leaving the electrode.

**Testing invention embodiments and results.** The effects of the present invention are demonstrated through the experimental results and graphs. The functionalized proteins from Table 1 were employed in the fabrication of electrochemical biosensors. To compare and showcase the applicability of our proposed methods for biosensors, we present several experimental results involving typical examples of bioelectrodes. These examples are based on two different mechanisms: direct electron transfer from the functionalized protein to the electrode (oxygen-independent, as shown in Figure 2a) and bi-enzymatic hydrogen peroxide detection (oxygen-dependent, as shown in Figures 2b). These mechanisms are integral components of this invention.

All the utilized oxidases, which catalyze the oxidation of C-N and C-O bonds using molecular oxygen, leading to the production of hydrogen peroxide, are enumerated in Table 1. As a result, three types of bioelectrodes are presented:
Type 1 The bioelectrodes based on a self-assembled monolayer (SAM) of functionalized protein (Figure 2a)
Type 2 The bioelectrodes based on a SAM of functionalized peptide with an additional deposited layer of non-functionalized protein (Figure 2b)

These various configurations illustrate the versatility of the proposed approach in utilizing different enzyme combinations for enhanced biosensor functionality.

The present invention introduces a biosensor designed as an electronic measurement device encompassing multiple functional bioelectrodes (Figure 3), serving the purpose of analyzing intricate analytes. Specifically, for the analysis of milk and its derivatives, the bioelectrodes built upon galactose oxidase, and glucose oxidase, and these enzymes in combination with lactase as well as lactate oxidase, can be effectively employed to detect galactose, glucose, lactose, and lactate within the same sample. Such an analysis facilitates the assessment of quality parameters and the freshness of milk.

In the context of complex human body fluids analysis, a network of bioelectrodes grounded in diverse enzymes like (T1-1) Peroxidase from horseradish, (T1-2) Glucose oxidase, (T1-3) Galactose oxidase and lactase, (T1-4) Cholesterol oxidase and cholesterol esterase, (T1-5) Alcohol oxidase, (T1-6) Monoamine oxidases A and B, (T1-7) Diamine oxidase, (T1-8) GABase, (T1-9) D-amine acid oxidase, (T1-10) L-amine acid oxidase, (T1-11) L-aspartate oxidase, (T1-12) L-glutamate oxidase, (T1-13) Lysine oxidase, (T1-13) Glycerol-3-phosphate oxidase, (T1-14) glycerol kinase, (T1-15) Coenzyme-A synthetase and acyl coenzyme-A oxidase, (T1-16) Acetylcholine esterase and choline oxidase, (T1-17) Oxalate oxidase, (T1-18) Sarcosine oxidase, (T1-19) Xanthine oxidase, (T1-20) Lactate oxidase, (T1-21) Pyruvate oxidase, (T1-22) Uricase, (T1-23) Myoglobin (Table 1), are synergistically employed to analyze corresponding analytes present within a single human body fluids sample. This comprehensive approach enables a profound insight into human body fluids composition.

For applications involving the monitoring of chemical processes, as well as the features and chemical dynamics of neuron interfaces in the brain and brain-computer interfaces, a range of bioelectrodes including functionalized peroxidase, glucose oxidase, monoamine oxidases A and B, diamino oxidase, GABAase, D-amino acid oxidase, L-glutamate oxidase, acetylcholine esterase in conjunction with choline oxidase, and lactate oxidase, can be harnessed to track selective fluctuations in neurotransmitter concentrations within the brain. These assays can effectively identify biomarkers as detailed in the SUMMARY OF THE INVENTION section.

The electrochemical biosensors operate on the foundation of a self-assembled monolayer (SAM) derived from functionalized peroxidase sourced from horseradish. These biosensors incorporate monoamine oxidases A and B, or acetylcholine esterase in conjunction with choline oxidase, along with xanthine oxidase. These tailored configurations are designed to facilitate the analysis of inhibitors targeting these specific enzymes. By harnessing enzyme inhibition as a principle, these electrochemical biosensors offer a practical, rapid, and environmentally conscious means of detecting inhibitors. This is particularly relevant for a range of substances, including natural compounds, antidepressant drugs, and compounds identified as chemical weapons. Thus, the innovation presented by the current invention extends to encompass the pharmaceutical and clinical evaluation of inhibitors; all facilitated through the use of electrochemical biosensing systems featuring multiple bioelectrodes (as illustrated in Figure 2b). In essence, this technology provides a robust platform for assessing enzyme inhibitors with diverse applications, contributing to both scientific research and practical analytical needs. Moreover, the versatility of the biosensors allows them to be adapted to various enzyme-based configurations for diverse applications, broadening the potential scope of this innovative technology.

The examples utilizing the glucose oxidase (GOx) enzyme and gold nanoparticles (AuNP) were chosen to demonstrate the distinctive characteristics of the bioelectrodes and their bioelectrocatalytic properties. The enzyme was bioconjugated with a phenothiazine (PTZ) group via electrochemical methods (Method C) in a mixture of acetonitrile and PPB buffer. Comparable outcomes were achieved using organic synthesis techniques (Methods A and B) within an organic solvent setting.

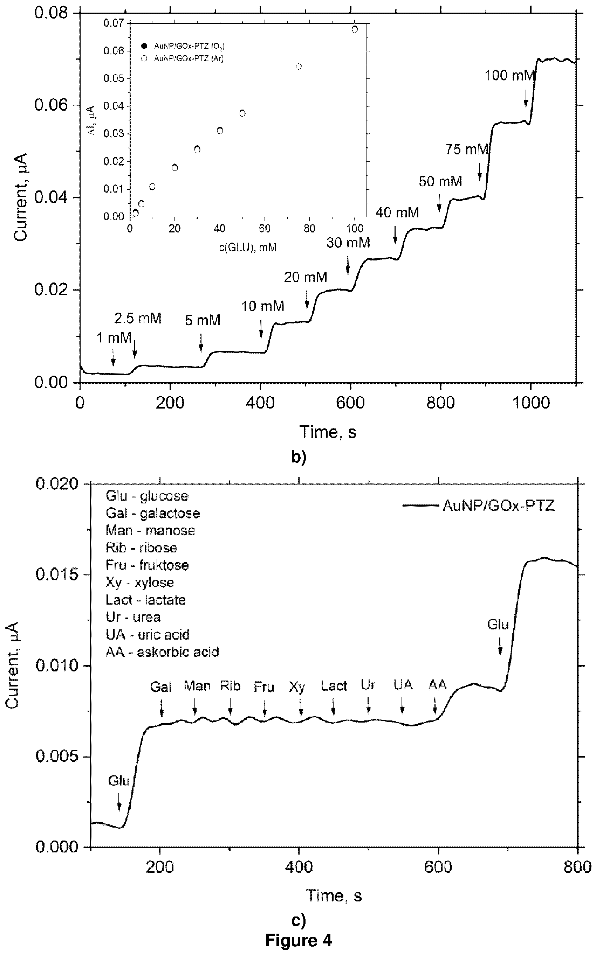

The functionalized protein was then immobilized as a self-assembled monolayer (SAM) onto an electrode that had been decorated with AuNPs to obtain the 1-type bioelectrode, as depicted in Figure 2a. To safeguard the adsorbed protein from being washed away, a dialysis membrane was employed as a protective barrier. In Figure 4a, bioelectrocatalytic currents at 0.2 V vs. Ag/AgCI were depicted when 100 mM glucose was introduced into the electrolyte. Notably, an increase in currents at 0.2 V vs. Ag/AgCI was observed with higher glucose concentrations, as shown in Figure 4b. The inset of Figure 4b demonstrates that the linear relationship between current and concentration is not constrained by the levels of glucose and oxygen present in the electrolyte, i.e., dissolved oxygen was eliminated by purging the electrolyte with argon gas. However, for the enzymes functionalized in water, the linearties of concentrations were observed only up to 20 mM of glucose **[1-5].** To evaluate the selectivity of this bioelectrode, potential interfering agents commonly found in human body fluids or food samples, including galactose, mannose, ribose, fructose, xylose, lactate, urea, uric acid, and ascorbic acid, were tested at a concentration of 1 mM in the test electrolyte. Figure 4c illustrates that these interfering agents did not significantly impede or hinder glucose detection, except for the minor impact of ascorbic acid.

The 1-type bioelectrodes created using similar protein functionalization methods exhibit comparable bioelectrocatalytic properties with corresponding substrates (analytes). For instance, employing a similar approach, a bioelectrode based on Horseradish peroxidase (I-type), functionalized in organic solvents with a ferrocene (HRP-Fc) group, was established onto a nanostructured electrode. This bioelectrode demonstrated the reduction of hydrogen peroxide at -0.2 V vs. Ag/AgCI in an electrolyte without the need for a mediator, achieved through a DET mechanism.

The 1-type bioelectrode (Fig. 2a) without a membrane was utilized as a bimodal memristor as molecular-level logic element. The elements were connected in a circuit and used employing the electrochemical microscopy or conductive atomic force microscopy probes. The ON and OFF states are realized at the redox potential of the mediator onto protein and 0 V versus Ag/AgCI in the presence of the substrate or protons, respectively. An increase in the current density is observed here after switching on. A substrate is an energy source. The memristor does not work in the absence of a substrate.

The incorporating enzymes (oxidases) that produce hydrogen peroxide as a product can be integrated into these 2 and 3 types of bioelectrodes (as depicted in Figures 2b). Figure 2b showcases the oxidase solution (the protein concentration is around 1 mg mL⁻¹) being deposited onto the bioelectrode with a SAM of functionalized peroxidase, followed by drying.

Figure 5a demonstrates that at a reduction potential of -0.2 V vs. Ag/AgCI, the cathodic current decreases as the glucose concentration is elevated from 50 µM to 1 mM. This electrochemical signal is directly linked to the concentration of a product resulting from the enzymatic reaction, such as hydrogen peroxide. A reduction in currents at -0.2 V vs. Ag/AgCI is discerned as glucose concentration increases (Figure 5b). The inset of Figure 5b indicates that the linear relationship between current and concentration is influenced by the concentration of oxygen in the electrolyte. Notably, no signal was observed when dissolved oxygen was eliminated by purging the electrolyte with argon gas. To evaluate the selectivity of this bioelectrode, interfering substances commonly present in human body fluids or food samples (like ascorbic acid, a mixture of amino acids, urea, cholesterol, and uric acid), as well as other carbohydrates (such as galactose, mannose, ribose, lactose, fructose, xylose, sucrose, and maltose), were tested at a concentration of 1 mM.

Figure 5c indicates that these substances did not significantly interfere with or inhibit glucose detection, except for specific cases. This type of biosensor scheme is suitable for all enzymes that either generate hydrogen peroxide or consume oxygen.

### NON-PATENT LITERATURE CITATIONS LIST

**[1].** Albery, W.J., Bartlett, P.N., Craston, D.H., 1985. Amperometric enzyme electrodes: Part II. Conducting salts as electrode materials for the oxidation of glucose oxidase. J. Electroanal. Chem. Interfacial Electrochem. 194, 223-235. https://doi.org/10.1016/0022-0728(85)85006-3
**[2].** Badia, A., Carlini, R., Fernandez, A., Battaglini, F., Mikkelsen, S.R., English, A.M., 1993. Intramolecular electron-transfer rates in ferrocene-derivatized glucose oxidase. J. Am. Chem. Soc. 115, 7053-7060. https://doi.org/10.1021/ja00069a001
**[3].** Degani, Y., Heller, Adam., 1987. Direct electrical communication between chemically modified enzymes and metal electrodes. I. Electron transfer from glucose oxidase to metal electrodes via electron relays, bound covalently to the enzyme. J. Phys. Chem. 91, 1285-1289. https://doi.org/10.1021/j100290a001
**[4].** Degani, Yinon., Heller, Adam., 1988. Direct electrical communication between chemically modified enzymes and metal electrodes. 2. Methods for bonding electron-transfer relays to glucose oxidase and D-amino-acid oxidase. J. Am. Chem. Soc. 110, 2615-2620. https://doi.org/10.1021/ja00216a040
**[5].** Krikstopaitis, K., Kulys, J., Tetianec, L., 2004. Bioelectrocatalytical glucose oxidation with phenoxazine modified glucose oxidase. Electrochem. Commun. 6, 331-336. https://doi.org/10.1016/j.elecom.2004.01.011
**[6].** Dudkaite, V., Kairys, V., Bagdži 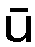 nas, G., 2023. Understanding the activity of glucose oxidase after exposure to organic solvents. J. Mater. Chem. B 11, 2409-2416. https://doi.org/10.1039/D2TB02605H
**[7].** Dudkaite, V., Bagdži 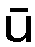 nas, G., 2022. Functionalization of Glucose Oxidase in Organic Solvent: Towards Direct Electrical Communication across Enzyme-Electrode Interface. Biosensors 12, 335. https://doi.org/10.3390/bios12050335
[8]. Bagdži 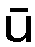 nas, G., Ramanavičius, A., 2019. Towards direct enzyme wiring: a theoretical investigation of charge carrier transfer mechanisms between glucose oxidase and organic semiconductors. Phys. Chem. Chem. Phys., 2019, 21, 2968-2976. https://doi.org/10.1039/C8CP07233G
[9]. Kommoju, P.-R., Chen, Z., Bruckner, R.C., Mathews, F.S., Jorns, M.S., 2011. Probing Oxygen Activation Sites in Two Flavoprotein Oxidases Using Chloride as an Oxygen Surrogate. Biochemistry 50, 5521-5534. https://doi.org/10.1021/bi200388g

## Claims

1. A biosensitive protein with catalytic properties, said biosensitive protein being functionalized with a substituted functional group, **wherein** a precursor of said functional group attached or included to said protein is water-insoluble or slightly-soluble in the range of 0.1 to 1.0 mg mL⁻¹, and the biosensitive protein is stable in an organic solvent, and said precursor of the functional group is soluble in said organic solvent, wherein said biosensitive protein is functionalized with a substituted functional group in said organic solvent.

2. The protein according to claim 1, **wherein** said protein is selected from a group consisting of: (T1-1) Peroxidase from horseradish, (T1-2) Glucose oxidase, (T1-3) Galactose oxidase and lactase, (T1-4) Cholesterol oxidase and cholesterol esterase, (T1-5) Alcohol oxidase, (T1-6) Monoamine oxidases A and B, (T1-7) Diamine oxidase, (T1-8) GABase, (T1-9) D-amine acid oxidase, (T1-10) L-amine acid oxidase, (T1-11) L-aspartate oxidase, (T1-12) L-glutamate oxidase, (T1-13) Lysine oxidase, (T1-14) Glycerol-3-phosphate oxidase, (T1-14) glycerol kinase, (T1-15) Coenzyme-A synthetase and acyl coenzyme-A oxidase, (T1-16) Acetylcholine esterase and choline oxidase, (T1-17) Oxalate oxidase, (T1-18) Sarcosine oxidase, (T1-19) Xanthine oxidase, (T1-20) Lactate oxidase, (T1-21) Pyruvate oxidase, (T1-22) Uricase, (T1-23) Myoglobin.

3. The protein according to any one of claims 1 to 2, **wherein** a functional group precursor used to functionalize said protein is selected a group consisting of: (T2-1) ferrocene, (T2-2) carbazole, (T2-3) phenothiazine, (T2-4) phenoselenazine, (T2-5) phenoxazine, (T2-6) acridane, (T2-7) thianthrene, (T2-8) selenanthrene, (T2-9) anthraquinone, (T2-10) fullerene, (T2-11) perylene tetracarboxylic diimide.

4. A biorecognition device **comprising** a bioreactive element comprising the functionalized biosensitive protein according to any of claims 1 to 3.

5. The device according to claim 4, **which** is a bioelectrode further comprising:
• an electrode (6) of any one: gold, platinum, gold, silver, copper, aluminium, iridium, palladium, platinum, rhodium, silicon, zinc, iron, steel, indium tin oxide and fluorine-doped tin oxide glass, graphite, and graphene conducting substrate with or without the gold (Au), silver (Ag), platinum (Pt), alumina (Al₂O₃), zinc oxide (ZnO) and silica (SiO₂) nanoparticles,
• and an outer analyte-permeable membrane (5), wherein the biosensitive functionalized protein is a layer (3) immobilized on the electrode (6) and covered by said outer analyte-permeable membrane (5).

6. The bioelectrode according to claim 5, further **comprising** an additional layer (1) of a same non-functionalized protein deposited between the analyte-permeable membrane (5) and the layer of the functionalized protein (3).

7. The device according to claim 4, **which** is a bimodal memristor **comprising:**
• a substrate of any one: gold, platinum, gold, silver, copper, aluminium, iridium, palladium, platinum, rhodium, silicon, zinc, iron, steel, indium tin oxide and fluorine-doped tin oxide glass, graphite, and graphene conducting substrate with or without the gold, silver, platinum, alumina, zinc oxide and silica nanoparticles;
• a layer of the functionalized protein, immobilized onto the substrate;
**wherein** the substrate is as an energy source and a circuit closing reagent, and the both layers of the substrate and the functionalized protein are arranged to be connected into an electrical circuit.

8. A biosensor, comprising at least one or more bioelectrodes according to any of claims 5 to 6, configured to measure one or more bioreactive analytes provided to the biosensor.

9. The biosensor according to claim 8, wherein said biosensor comprises more than one bioelectrodes, **wherein** said more than one bioelectrodes comprise more than one different biosensitive proteins with catalytic properties, whereby the biosensor is configured to measure simultaneously concentrations of more than one bioreactive analytes provided into the biosensor.

10. The biosensor according to any of claims 8 to 9, **wherein** said biosensor device is configured to detect bioanalytes selected from a group consisting of: (T1-1a) hydrogen peroxide, dopamine, oxygen (T1-2a) Glucose, (T1-3a) Galactose, lactose, (T1-4a) Cholesterol, (T1-5a) Ethanol, Methanol, (T1-6a) Serotonin, Adrenaline, Noradrenaline, Nicotine or other inhibitors of these enzymes, (T1-7a) Adrenaline, Noradrenaline, (T1-8a) γ-Aminobutyric acid (GABA), (T1-9a) D-amino acids, D-Serine, D-Lysine, Serotonin, (T1-10a) L-Amino acids, (T1-11a) L-Aspartate, (T1-12a) L-Glutamate, L-Glutamide, (T1-13a) L-Lysine, (T1-14a) Glycerol, (T1-15a) Free fatty acids, peroxisomal acetyl-CoA, (T1-16a) Acetylcholine, organophosphate insecticides, chemical weapon compounds as acetylcholine esterase inhibitors, (T1-17a) Oxalate, (T1-18a) Sarcosine, (T1-19a) Xanthine, (T1-20a) Lactate, (T1-21a) Pyruvate, (T1-22a) Uric acid, (T1-23a) Oxygen, nitrogen(II) oxide.

11. The electrochemical biosensor according to any of claims of 8 and 10 for enzyme inhibition-based biosensors with the substrate of the corresponding inhibited enzyme.

12. A biochemosensoric computer-brain interface, comprising the biosensor according to any of claims 8 to 11.

13. A method of producing the biosensitive functionalized protein according to any of claims 1 to 3, **wherein** the functionalization of the biosensitive protein with the functional group is performed in organic solvents other than chloroform, dimethylformamide and dimethyl sulfoxide.

14. The protein functionalization method according to claim 13, **wherein** the organic solvents for functionalization of the biosensitive protein with the functional group, is selected from a group consisting of: dichlormethane, 1,2-dichlorethane, tetrahydrofuran, 1,2-dimethoxyethane, toluene ethylacetate, acetonitrile, ethanol, and preferably, from tetrahydrofuran, acetonitrile.

15. The method according to any of claims 13-14, **comprising** steps of:
• selecting a biosensitive protein, selecting a functional group precursor, and dissolving both in a selected organic solvent;
• stirring the obtained mixture for 24-48 hours;
• evaporating from the mixture the organic solvent at the temperature of the bath not exceeding 40 °C, for example, using a rotary evaporator;
• dissolving the residue obtained after the evaporation, in a potassium phosphate, or acetate, or citric buffer solution;
• filtering the buffer solution to remove not dissolved substances;
• extracting the excess of functional material from the filtered solution with chlorinated organic solvent to obtain a pure product.

## Amended claims

### Amended claims in accordance with Rule 137(2) EPC.

1. A method of functionalizing a biosensitive protein with catalytic properties, said biosensitive protein functionalized with a substituted functional group, **wherein**
- a precursor of said functional group attached or included to said biosensitive protein is water-insoluble or slightly-soluble in the range of 0.1 to 1.0 mg mL⁻¹, and
- the biosensitive protein is stable in an organic solvent, wherein the biosensitive protein is selected from a group consisting of: (T1-1) peroxidase from horseradish, (T1-2) glucose oxidase, and
- said precursor of the functional group is soluble in said organic solvent, wherein said organic solvent excludes any of: chloroform, dimethylformamide (DMF), and dimethyl sulfoxide (DMSO),
- wherein said biosensitive protein is functionalized with a substituted functional group in said organic solvent.

2. The protein functionalization method according to claim 1, **wherein** a functional group precursor used to functionalize said protein is selected a group consisting of: (T2-1) ferrocene, (T2-2) carbazole, (T2-3) phenothiazine, (T2-4) phenoselenazine, (T2-5) phenoxazine, (T2-6) acridane, (T2-7) thianthrene, (T2-8) selenanthrene, (T2-9) anthraquinone, (T2-10) fullerene, (T2-11) perylene tetracarboxylic diimide.

3. The protein functionalization method according to any of claims 1 to 2, **wherein** the organic solvents for functionalization of the biosensitive protein with the functional group, is selected from a group consisting of: dichlormethane, 1,2-dichlorethane, tetrahydrofuran, 1,2-dimethoxyethane, toluene ethylacetate, acetonitrile, ethanol, and preferably, from tetrahydrofuran, acetonitrile.

4. The protein functionalization method according to any of claims 1 to 3, **comprising** steps of:
• selecting a biosensitive protein, selecting a functional group precursor, and dissolving both in a selected organic solvent;
• stirring the obtained mixture for 24-48 hours;
• evaporating from the mixture the organic solvent at the temperature of the bath not exceeding 40 °C, for example, using a rotary evaporator;
• dissolving the residue obtained after the evaporation, in a buffer solution;
• filtering the buffer solution to remove not dissolved substances;
• extracting the excess of functional material from the filtered solution with chlorinated organic solvent to obtain a pure product.

5. A functionalized biosensitive protein, obtainable according to any of claims 1 to 4.

6. A biorecognition device **comprising** a bioreactive element comprising the functionalized biosensitive protein obtainable according to any of claims 1 to 4.

7. The biorecognition device according to claim 6, **which** is a bioelectrode further comprising:
• an electrode (6) of any one: gold, platinum, gold, silver, copper, aluminium, iridium, palladium, platinum, rhodium, silicon, zinc, iron, steel, indium tin oxide and fluorine-doped tin oxide glass, graphite, and graphene conducting substrate with or without the gold (Au), silver (Ag), platinum (Pt), alumina (Al₂O₃), zinc oxide (ZnO) and silica (SiO₂) nanostructures,
• and an outer analyte-permeable membrane (5), wherein the biosensitive functionalized protein is a layer (3) immobilized on the electrode (6) and covered by said outer analyte-permeable membrane (5).

8. The bioelectrode according to claim 7, further **comprising** an additional layer (1) of a non-functionalized protein deposited between the analyte-permeable membrane (5) and the layer of the functionalized protein (3).

9. The biorecognition device according to claim 6, **which** is a memristor **comprising**:
• a substrate of any one: gold, platinum, gold, silver, copper, aluminium, iridium, palladium, platinum, rhodium, silicon, zinc, iron, steel, indium tin oxide and fluorine-doped tin oxide glass, graphite, and graphene conducting substrate with or without the gold, silver, platinum, alumina, zinc oxide and silica nanostructures;
• a layer of the functionalized protein, immobilized onto the substrate;
**wherein** the substrate is as an energy source and a circuit closing reagent, and the both layers of the substrate and the functionalized protein are arranged to be connected into an electrical circuit.

10. A biosensor, comprising at least one or more bioelectrodes according to any of claims 7 to 9, configured to measure one or more bioreactive analytes provided to the biosensor.

11. The biosensor according to claim 10, wherein said biosensor comprises more than one bioelectrodes, **wherein** said more than one bioelectrodes comprise more than one different biosensitive proteins with catalytic properties, whereby the biosensor is configured to measure simultaneously concentrations of more than one bioreactive analytes provided into the biosensor.

12. The biosensor according to claim 8 with any of claims 10 to 11, **wherein** said biosensor device comprises the layer of the non-fuctionalized biosensitive protein selected from a group consisting of: (T1-1) peroxidase from horseradish, (T1-2) glucose oxidase, galactose oxidase and lactase, (T1-4) cholesterol oxidase and cholesterol esterase, (T1-5) alcohol oxidase, (T1-6) monoamine oxidases A and B, (T1-7) diamine oxidase, (T1-8) GABase, (T1-9) D-amine acid oxidase, (T1-10) L-amine acid oxidase, (T1-11) L-aspartate oxidase, (T1-12) L-glutamate oxidase, (T1-13) lysine oxidase, (T1-14) glycerol-3-phosphate oxidase, (T1-14) glycerol kinase, (T1-15) coenzyme-A synthetase and acyl coenzyme-A oxidase, (T1-16) acetylcholine esterase and choline oxidase, (T1-17) oxalate oxidase, (T1-18) sarcosine oxidase, (T1-19) xanthine oxidase, (T1-20) lactate oxidase, (T1-21) pyruvate oxidase, (T1-22) uricase, (T1-23) myoglobin,
whereby the biosensor device is configured to detect bioanalytes selected from a group consisting of: (T1-1a) hydrogen peroxide, dopamine, oxygen (T1-2a) glucose, (T1-3a) galactose, lactose, (T1-4a) cholesterol, (T1-5a) ethanol, methanol, (T1-6a) serotonin, adrenaline, noradrenaline, nicotine or other inhibitors of these enzymes, (T1-7a) adrenaline, noradrenaline, (T1-8a) γ-aminobutyric acid (GABA), (T1-9a) D-amino acids, D-Serine, D-Lysine, serotonin, (T1-10a) L-amino acids, (T1-11a) L-aspartate, (T1-12a) L-glutamate, L-glutamide, (T1-13a) L-lysine, (T1-14a) glycerol, (T1-15a) free fatty acids, peroxisomal acetyl-CoA, (T1-16a) acetylcholine, organophosphate insecticides, chemical weapon compounds as acetylcholine esterase inhibitors, (T1-17a) oxalate, (T1-18a) sarcosine, (T1-19a) xanthine, (T1-20a) lactate, (T1-21a) pyruvate, (T1-22a) uric acid, (T1-23a) oxygen, nitrogen(II) oxide.

13. The electrochemical biosensor according to any of claims of 10 and 12 for enzyme inhibition-based biosensors with the substrate of the corresponding inhibited enzyme.

14. A biochemosensoric computer-brain interface, comprising the biosensor according to any of claims 10 to 13.
